# FASCICULE DE BREVET EUROPEEN

(11) **EP 0 783 883 B1**
(45) Date de publication et mention de la délivrance du brevet: **14.07.1999**
(21) Numéro de dépôt: 96402614.0
(22) Date de dépôt: 03.12.1996
(51) Int. Cl.: A61K 7/02, A61K 7/06, A61K 7/48

(54) **Utilisation d'un polyholoside dans une composition nettoyante ou démaquillante, et composition le comprenant**
Verwendung eines Polyholosids in einer Reinigungs- oder Abschmink-Zusammensetzung und Zusammensetzung, die ein Polyholisid enthält
Use of polyholosides in cleaning compositions or in make-up removers, and compositions containing the same

(30) Priorité: 10.01.1996 FR 9600222; 21.05.1996 FR 9606287; 09.07.1996 FR 9608537
(43) Date de publication de la demande: 16.07.1997
(73) Titulaire: L'OREAL, 75008 Paris (FR)
(72) Inventeur: Vanstraceele, Anne, 75013 Paris (FR); Marion, Catherine, 92330 Sceaux (FR)
(74) Mandataire: Dodin, Catherine

(56) Documents cités:
- EP-A- 0 285 829
- EP-A- 0 651 990
- WO-A-92/06778
- WO-A-93/00067
- WO-A-93/07855
- FR-A- 2 609 397
- GB-A- 2 250 998
- US-E- R E28 781

## Description

La présente invention se rapporte à l'utilisation d'un polyholoside dans une composition notamment cosmétique destinée au nettoyage et/ou au démaquillage de la peau et/ou des muqueuses et/ou des yeux.

Il est connu d'utiliser des huiles démaquillantes dans des compositions pour le démaquillage de la peau et/ou des yeux. Généralement, ces compositions comprennent également des tensioactifs qui permettent d'améliorer l'élimination de ladite huile et/ou des traces de maquillage. Or, on sait que l'utilisation de ces compositions peut laisser une impression d'inconfort, notamment sur les paupières et/ou sur les yeux.

Il est également connu des compositions de démaquillage à base uniquement de tensioactifs, qui vont permettre l'élimination des traces de maquillage tout en facilitant la mise en émulsion de la composition, en rendant compatible les phases grasse et aqueuse de la composition lorsqu'elle se présente sous la forme d'une émulsion. Toutefois, les tensioactifs peuvent présenter l'inconvénient d'être plus ou moins irritants pour la peau. Pour éviter l'utilisation de tensioactifs, le document WO-A-93/07855 décrit une composition de nettoyage à base d'argile de type hectorite.

Le document EP-A-285829 décrit un procédé de préparation d'un hétéropolysaccharide acide par culture de tissus végétaux. Le document WO-A-92/06778 se rapporte à des émulsions à base d'alkylpolyholosides.

L'invention a pour but de pallier les inconvénients de l'art antérieur et de proposer une composition qui permet un nettoyage et/ou un démaquillage facile et adéquat, qui n'est pas irritante pour la peau et/ou les yeux, et qui ne contient pas de tensioactif.

L'invention a donc pour premier objet une composition pour le nettoyage et/ou le démaquillage de la peau et/ou des muqueuses et/ou des yeux, comprenant un polyholoside homogène et/ou un composé qui comprend un polyholoside homogène.

Un autre objet de l'invention est l'utilisation de polyholoside homogène et/ou d'un composé le comprenant dans une composition de nettoyage et/ou de démaquillage pour la peau et/ou les muqueuses et/ou les yeux, comprenant un support cosmétiquement ou dermatologiquement acceptable, notamment en tant qu'agent nettoyant et/ou démaquillant, et/ou afin d'obtenir une composition démaquillante peu ou pas irritante, et/ou afin d'obtenir une composition présentant un toucher doux, et/ou afin d'obtenir une composition gélifiée.

Un autre objet de l'invention est un procédé de nettoyage et/ou de démaquillage de la peau et/ou des muqueuses et/ou des yeux, caractérisé en ce qu'on applique sur la peau et/ou sur les muqueuses et/ou sur les yeux une composition telle que définie ci-dessus.

On a en effet constaté que l'utilisation d'un polyholoside selon l'invention permet d'obtenir une composition nettoyante et/ou démaquillante efficace, non irritante et peu collante, qui présente de surcroît un toucher doux et agréable.

De plus, l'incorporation d'un polyholoside dans des compositions, notamment cosmétiques, permet l'obtention d'une composition gélifiée sans ajout supplémentaire d'agent gélifiant classiquement utilisé. Le gel obtenu est lisse et onctueux.

La présente invention a donc pour objet l'utilisation d'un polyholoside homogène en tant qu'agent démaquillant, et/ou dans une composition démaquillante.

Les saccharides, de formule Cₙ(H₂O)ₙ, sont généralement divisés en deux catégories : les oses ou sucres simples, et les osides ou association de plusieurs molécules. Parmi les osides, on peut distinguer les holosides qui sont formés uniquement de sucres et les hétérosides qui sont constitués par un ou plusieurs oses et une partie non glucidique. Parmi les polyholosides, on peut encore distinguer les polyholosides homogènes qui résultent de l'association d'un même ose, et les polyholosides hétérogènes qui résultent soit de l'association d'oses différents, soit de l'association d'oses ayant la même formule chimique brute mais de configuration géométrique différente (isomères D et L par exemple).

Ainsi, les polyholosides selon l'invention sont homogènes, donc sont constitués uniquement de sucres et résultent de l'association d'un même ose. Ils peuvent toutefois être substitués, notamment par des chaînes grasses ou des groupements aminés.

Les polyholosides selon l'invention peuvent comprendre principalement 2 à 10 fois le même ose (oligoholosides), ou plus de 10 fois le même ose.

Les polyholosides selon l'invention peuvent comprendre principalement un motif ose unique, choisi parmi tous les motifs oses envisageables, d'origine naturelle ou synthétique, et notamment tels que :
- les aldoses comme les pentoses (ribose, arabinose, xylose ou apiose, par exemple) ou les hexoses (glucose, fucose, mannose ou galactose, par exemple),
- les cétoses tels que le fructose,
- les désoxyoses, tels que le rhamnose, le digitoxose, le cymarose ou l'oléandrose,
- les dérivés d'ose tels que les acides uroniques comme les acides mannuronique, guluronique, galacturonique ou glycuronique, ou encore les itols comme le mannitol ou le sorbitol.

Parmi les polyholosides selon l'invention, on peut citer :
- la chitine et ses dérivés, tels que les dérivés de chitosane, et notamment le lactate de chitosane, qui un polyholoside N-substitué;
- les dérivés de l'acide hyaluronique, tels que les sels métalliques, et notamment le hyaluronate de sodium qui est un polyholoside N-substitué;
- le dextrane et ses dérivés, tels que le sulfate de dextrane,
- la cellulose et ses dérivés, tels que la gomme de cellulose, l'hydroxyéthylcellulose, l'hydroxypropylcellulose, les polyquaternium 10 et 24;
- l'amidon, l'amylose et l'amylopectine et leurs dérivés;
- le chondroïde et ses dérivés, tels que le chondroïtine et le sulfate de chondroïtine.

On peut également utiliser des composés qui contiennent des polyholosides selon l'invention, notamment des composés naturels tels que le miel qui comprend environ 10% de dextrine.

Le polyholoside selon l'invention peut être éventuellement ramifié ou linéaire. Il peut également être substitué, par exemple par des chaînes grasses, notamment comprenant 8 à 30 atomes de carbone, et/ou par des groupements aminés.

Dans le cadre de la présente invention, on peut utiliser un polyholoside homogène seul, ou un mélange de polyholosides homogènes.

Les polyholosides selon l'invention sont de préférence introduits dans la composition sous forme de solution aqueuse qui peut comprendre 0,01 à 5% en poids de polyholoside.

Le polyholoside peut être présent dans la composition finale en une quantité de 0,01 à 5% en poids, de préférence 0,02 à 1% en poids.

Les polyholosides selon l'invention peuvent donc être utilisés en tant qu'agent démaquillant, en particulier dans une composition pour le nettoyage et/ou démaquillage de la peau du corps ou du visage, des muqueuses telles que les lèvres, et/ou des yeux.

Ils peuvent ainsi remplacer tout ou partie des tensioactifs généralement présents dans les compositions de nettoyage et/ou démaquillage de l'art antérieur.

Ladite composition peut se présenter sous la forme d'une émulsion, notamment huile-dans-eau ou eau-dans-huile, voire sous la forme d'une émulsion multiple.

Elle peut également se présenter sous forme d'une solution aqueuse, éventuellement gélifiée, ou sous forme d'une lotion, par exemple biphasée, d'une crème, d'un lait, voire d'une mousse.

La composition selon l'invention peut comprendre une phase huileuse à base d'huile animale, végétale, minérale, siliconée, fluorée et/ou synthétique.

La phase huileuse peut également comprendre des alcools gras ou des acides gras, ainsi que des tensioactifs et des émulsionnants.

On peut notamment citer les huiles hydrocarbonées telles que l'huile de paraffine ou de vaseline; le perhydrosqualène; l'huile d'arachide; l'huile d'amande douce de calophyllum, de palme, de ricin, d'avocat, de jojoba, d'olive ou de germes de céréales; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones.

La phase huileuse peut également comprendre une huile démaquillante telle qu'un ester d'acides gras, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone.

Un tel ester peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.

La phase huileuse peut être présente à raison de 5-95% en poids dans le cas d'une émulsion.

La composition selon l'invention peut comprendre, en outre,
- un agent permettant la mise en suspension de la phase grasse, par exemple un copolymère d'acrylates d'alkyle en C₁₀-C₃₀ et d'acide acrylique ou méthacrylique ou de leur ester (Pemulen TR1, Pemulen TR2, Carbopol 1342 de GOODRICH); ou un copolymère acrylamide/acide méthylpropane sulfonique (Sepigel de SEPPIC), et/ou
- un agent de mise en dispersion de la phase grasse, tel qu'un système émulsionnant ou vésiculaire à base de vésicules, éventuellement de taille nanométrique, constituées de lipides ioniques (liposomes) ou non ioniques, et en particulier les systèmes émulsionnants bien connus de l'homme du métier constitués de stéarate de glycéryl /PEG 100 stéarate (CTFA), d'alcool cétylique et d'alcool stéarylique.

La composition de l'invention peut comprendre, en outre, un agent pour modifier sa viscosité, et obtenir des textures plus ou moins gélifiées, tel que :
- les dérivés cellulosiques (carboxyméthylcellulose, hydroxyéthylcellulose, hydroxypropylméthylcellulose),
- les gommes naturelles telles que les gommes de xanthane, de guar, de caroube les scléroglucanes, les dérivés de chitine ou de chitosane, les carraghénanes,
- les dérivés polycarboxyvinyliques du type Carbomer (vendues par GOODRICH sous les dénominations Carbopol, 940, 951, 980, ou par 3V-SIGMA sous la dénomination Synthalen K ou Synthalen L).

La composition selon l'invention peut également comprendre, de façon connue des adjuvants couramment utilisés dans le domaine considéré, tels que les conservateurs, les antioxydants, les parfums, les charges telles que le kaolin ou l'amidon, voire les microsphères creuses, les pigments, les filtres UV, les séquestrants, les huiles essentielles, les matières colorantes, les actifs hydrophiles ou lipophiles tels que les hydratants, notamment la glycérine, le butylène glycol, les anti-inflammatoires comme l'allantoïne, le bisabolol, les anti-radicaux libres comme la vitamine E ou ses dérivés, les apaisants tels que l'eau de bleuet, l'extrait d'ris, les dépigmentants, les actifs biologiques tels que l'urée, les acides aminés, les vitamines et leurs dérivés, les protéines, l'acide salicylique et ses dérivés, les α-hydroxy-acides, l'acide pyrrolidone carboxylique et ses sels, les céramides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition présente de préférence un pH qui respecte la peau, généralement compris entre 5 et 8, de préférence un pH de 5,5 à 7,5.

La composition selon l'invention peut être conditionnée dans tout type d'article de conditionnement.

En particulier, les compositions de nettoyage et/ou démaquillage selon l'invention peuvent être avantageusement conditionnées en conditionnement mono-dose (ou uni-dose), à savoir un conditionnement tel que son contenu peut être utilisé en totalité lors d'un usage unique.

Un tel conditionnement peut en particulier être obtenu selon l'état de la technique, notamment par extrusion-soufflage-moulage d'une matière plastique.

Ce procédé permet donc à un contenant d'être moulé par soufflage, rempli avec la composition, puis scellé, en une seul opération aseptique et continue.

Dans une première étape, le polymère, notamment sous forme de granulé, peut être extrudé, ce qui permet également de le stériliser. Un dispositif de moulage se présentant sous forme de deux moitiés, peut venir se refermer sur le polymère extrudé pour former le corps du contenant. On peut alors injecter à l'intérieur du moulage un gaz tel que de l'air filtré stérile, ce qui va permettre de 'souffler' le contenant selon la forme du moule. On peut ensuite remplir le conditionnement ainsi obtenu avec la composition de nettoyage selon l'invention, ladite composition étant de manière préférée préalablement stérilisée. Quand le volume requis est rempli, on peut fermer le conditionnement. Un tel procédé est notamment décrit dans l'articule 'Sterility and versatility in cosmetic cream packaging' de D. Wilson, paru dans Cosmetics and Toiletries Manufacture Worlwide, pages 253-256.

Un autre objet de l'invention est donc une composition pour le nettoyage et/ou le démaquillage de la peau et/ou des muqueuses et/ou des yeux, comprenant un polyholoside homogène, caractérisée par le fait qu'elle est conditionnée en monodose.

L'invention est illustrée plus en détail dans les exemples suivants.

### Exemple 1 : Lotion démaquillante pour les veux

| | |
|---|---|
| . lactate de chitosane | 0,5 g |
| . allantoine | 0,1 g |
| . eau de bleuet | 3 g |
| . eau de rose | 0,5 g |
| . conservateur | qs |
| . eau | qsp 100 g |

Cette lotion, qui ne contient pas de tensioactif, permet un démaquillage efficace des yeux; de plus, elle convient particulièrement aux yeux sensibles.

### Exemple 2 : Lotion démaquillante pour les veux

| | |
|---|---|
| . hyaluronate de sodium | 0,05 g |
| . allantoine | 0,1 g |
| . eau de bleuet | 3 g |
| . eau de rose | 0,5 g |
| . conservateur | qs |
| . eau | qsp 100 g |

Cette lotion, qui ne contient pas de tensioactif, permet un démaquillage efficace et convient particulièrement aux yeux sensibles.

### Exemple 3 : Lotion biphasée démaquillante

| *phase aqueuse (55% en poids)* | |
|---|---|
| . lactate de chitosane | 0,5 g |
| . sel de cuivre | 0,04 g |
| . conservateur | qs |
| . eau | qsp 100 g |

| *phase grasse (45% en poids)* | |
|---|---|
| . huile de vaseline | 15 g |
| . huile de silicone volatile | 55 g |
| . palmitate d'éthyle 2-hexyle | 30 g |

On obtient une lotion biphasée qui permet un démaquillage facile et efficace même pour un maquillage waterproof.

### Exemple 4 : Lait démaquillant pour le visage et les yeux

| | |
|---|---|
| . dextrane | 0,5 g |
| . palmitate d'éthyle 2-hexyle | 25 g |
| . glycérine | 3 g |
| . polymère carboxyvinylique | 0,5 g |
| . NaOH | 0,3 g |
| . conservateur | qs |
| . eau | qsp 100 g |

Cette composition se présente sous forme d'un lait démaquillant ayant de bonnes propriétés cosmétiques, et étant doux et confortable à utiliser.

### Exemple 5 : Lait démaquillant pour le visage et les yeux

| | |
|---|---|
| . hyaluronate de sodium | 0,05 g |
| . palmitate d'éthyle 2-hexyle | 25 g |
| . glycérine | 3 g |
| . polymère carboxyvinylique | 0,5 g |
| . NaOH | 0,3 g |
| . conservateur | qs |
| . eau | qsp 100 g |

Cette composition se présente sous forme d'un lait démaquillant ayant de bonnes propriétés cosmétiques, et étant doux et confortable à utiliser.

### Exemple 6 : Gel démaquillant pour le visage

| | |
|---|---|
| . hyaluronate de sodium | 0,05 g |
| . glycérine | 3 g |
| . Carbomer | 0,8 g |
| . beurre de karité | 1 g |
| . alcool | 5 g |
| . conservateur | qs |
| . eau | qsp 100 g |

Cette composition se présente sous forme d'un gel démaquillant ayant de bonnes propriétés cosmétiques.

### Exemple 7 : Crème démaquillante

| | |
|---|---|
| . hyaluronate de sodium | 0,01 g |
| . palmitate d'éthyle 2-hexyle | 10 g |
| . huile de silicone volatile | 10 g |
| . huile d'amande d'abricot | 7 g |
| . alcool stéarylique | 5 g |
| . stéarate de potassium | 0,5 g |
| . stéarate de sorbitane + cocoate de sucrose | 4 g |
| . gomme de xanthane | 0,3 g |
| . conservateur | qs |
| . eau | qsp 100 g |

Cette composition se présente sous forme d'une crème démaquillante ayant de bonnes propriétés cosmétiques.

### Exemple 8 : Lotion démaquillante pour les yeux

| | |
|---|---|
| . hyaluronate de sodium | 0,05 g |
| . allantoine | 0,1 g |
| . miel | 0,3 g |
| . poloxamer 184 | 1 g |
| . eau de bleuet | 3 g |
| . eau de rose | 0,5 g |
| . conservateur | qs |
| . eau | qsp 100 g |

Cette lotion, qui contient un tensioactif, permet un démaquillage efficace des yeux.

### Exemple 9

On prépare deux solutions aqueuses comprenant, l'une 0,5% en poids de lactate de chitosane, l'autre 0,05% en poids d'hyaluronate de sodium.
*Test 1* : on compare le pouvoir démaquillant de ces deux solutions, avec celui de l'eau et celui d'un démaquillant du commerce.
Le pouvoir démaquillant est déterminé à l'aide de la technique dite du "robot démaquilleur" :
   . l'appareil se compose d'une plaque et d'un bras muni d'un poids exerçant sur la plaque une pression de 100 g/cm² et équipé à l'une de ses extrémités d'un coton qui glisse sur la plaque.
   . on dépose sur la plaque une fine couche d'un mascara waterproof noir commercialisé sous la marque Keracils par Lancôme.
   . cette plaque est mise à sécher pendant 4 h.
   . on réalise ensuite 8 passages simples sans retour, d'un coton imbibé de 1 ml de composition à tester, le coton étant changé après chaque passage.
   . on évalue visuellement la quantité de mascara restant sur la plaque après les 8 passages.

   Les 8 passages correspondent à un démaquillage parfait avec le démaquillant du commerce, pris comme référence.
   On constate que le pouvoir démaquillant des deux solutions comprenant un polyholoside selon l'invention est supérieur à celui de l'eau, et égal voire supérieur à celui du démaquillant du commerce.
*Test 2* : ces résultats ont été confirmés par un test in vivo, réalisé sur 10 personnes.
   Les solutions selon l'invention sont testées en comparaison avec le démaquillant du commerce (témoin), pendant au moins 3 jours.
   Trois critères sont évalués : le pouvoir démaquillant, le caractère cosmétique (douceur, glissant, ...) et le confort.
   On obtient les résultats suivants :
   - solution comprenant le lactate de chitosane :
      . pouvoir démaquillant identique à celui du démaquillant témoin
      . caractère cosmétique identique à celui du démaquillant témoin
      . confort supérieur à celui du démaquillant témoin
   - solution comprenant le hyaluronate de sodium :
      . pouvoir démaquillant supérieur à celui du démaquillant témoin
      . caractère cosmétique supérieur à celui du démaquillant témoin
      . confort supérieur à celui du démaquillant témoin

## Revendications

1. Composition pour le nettoyage et/ou le démaquillage de la peau et/ou des muqueuses et/ou des yeux, comprenant un polyholoside homogène et/ou un composé qui comprend un polyholoside homogène choisi parmi la chitine et ses dérivés ; les dérivés de l'acide hyaluronique ; les dérivés du dextrane ; les polyquaternium 10 et 24 ; l'amylose et l'amylopectine et leurs dérivés ; le chondroïde et ses dérivés.

2. Composition selon la revendication 1, dans laquelle le polyholoside homogène est choisi parmi les dérivés de chitosane, les sels métalliques de l'acide hyaluronique, le sulfate de dextrane, le chondroïtine et le sulfate de chondroïtine.

3. Composition selon la revendication 1 ou 2, dans laquelle le polyholoside homogène est choisi parmi le lactate de chitosane, le hyaluronate de sodium.

4. Composition selon la revendication 1, dans laquelle ledit composé est du miel.

5. Composition selon l'une des revendications précédentes, dans laquelle le polyholoside est présent dans la composition finale en une quantité de 0,01 à 5% en poids.

6. Composition selon l'une des revendications précédentes, dans laquelle le polyholoside est présent dans la composition finale en une quantité de 0,02 à 1% en poids, par rapport à la composition.

7. Composition selon l'une des revendications précédentes, ne comprenant pas de tensioactif.

8. Composition selon l'une des revendications précédentes, se présentant sous la forme d'une émulsion, d'une solution aqueuse éventuellement gélifiée, d'une lotion notamment biphasée, d'une crème, d'un lait, d'une mousse.

9. Composition selon l'une des revendications précédentes, caractérisée par le fait qu'elle est conditionnée en monodose.

10. Utilisation d'au moins un polyholoside homogène et/ou d'au moins un composé comprenant un polyholoside homogène en tant qu'agent nettoyant et/ou démaquillant, dans une composition de nettoyage et/ou de démaquillage pour la peau et/ou les muqueuses et/ou les yeux, comprenant un support cosmétiquement ou dermatologiquement acceptable, le polyholoside étant choisi parmi la chitine et ses dérivés, tels que les dérivés de chitosane, et notamment le lactate de chitosane; les dérivés de l'acide hyaluronique, tels que les sels métalliques, et notamment le hyaluronate de sodium; le dextrane et ses dérivés, tels que le sulfate de dextrane; la cellulose, l'hydroxyéthylcellulose, l'hydroxypropylcellulose, les polyquaternium 10 et 24; l'amidon, l'amylose et l'amylopectine et leurs dérivés; le chondroïde et ses dérivés, tels que le chondroïtine et le sulfate de chondroïtine.

11. Utilisation selon la revendication 10, pour la préparation d'une composition démaquillante peu ou pas irritante, et/ou d'une composition présentant un toucher doux et/ou une composition gélifiée.

12. Utilisation selon l'une des revendications 10 à 11, dans laquelle ledit composé est du miel.

13. Utilisation selon l'une des revendications 10 à 12, dans laquelle le polyholoside est présent dans la composition finale en une quantité de 0,01 à 5% en poids, de préférence 0,02 à 1% en poids, par rapport à la composition.

14. Utilisation selon l'une des revendications 10 à 13, dans laquelle la composition ne comprend pas de tensioactif.

15. Utilisation selon l'une des revendications 10 à 14, dans une composition se présentant sous la forme d'une émulsion, d'une solution aqueuse, éventuellement gélifiée, d'une lotion notamment biphasée, d'une crème, d'un lait, d'une mousse.

16. Procédé de nettoyage et/ou de démaquillage de la peau et/ou des muqueuses et/ou des yeux, caractérisé en ce qu'on applique sur la peau et/ou sur les muqueuses et/ou sur les yeux une composition selon l'une quelconque des revendications 1 à 9.

## Claims

1. Composition for cleansing and/or removing makeup from the skin and/or mucosae and/or eyes, comprising a homogeneous polyholoside and/or a compound which comprises a homogeneous polyholoside chosen from chitin and its derivatives; hyaluronic acid derivatives; dextran derivatives; polyquaterniums 10 and 24; amylose and amylopectin and their derivatives; and chondroprotein and its derivatives.

2. Composition according to Claim 1, in which the homogeneous polyholoside is chosen from chitosan derivatives, metal salts of hyaluronic acid, dextran sulphate, chondroitin and chondroitin sulphate.

3. Composition according to Claim 1 or 2, in which the homogeneous polyholoside is chosen from chitosan lactate and sodium hyaluronate.

4. Composition according to Claim 1, in which the said compound is honey.

5. Composition according to one of the preceding claims, in which the polyholoside is present in the final composition in a quantity of from 0.01 to 5% by weight.

6. Composition according to one of the preceding claims, in which the polyholoside is present in the final composition in a quantity of from 0.02 to 1% by weight, relative to the composition.

7. Composition according to one of the preceding claims, which does not comprise any surfactant.

8. Composition according to one of the preceding claims, in the form of an emulsion, an optionally gelled aqueous solution, a lotion, especially a biphase lotion, a cream, a milk or a mousse.

9. Composition according to one of the preceding claims, characterized in that it is packaged in a single-dose pack.

10. Use of at least one homogeneous polyholoside and/or at least one compound comprising a homogeneous polyholoside as a cleansing and/or makeup-removing agent, in a cleansing and/or makeup remover composition for the skin and/or mucosae and/or eyes, comprising a cosmetically or dermatologically acceptable vehicle, the polyholoside being chosen from chitin and its derivatives, such as chitosan derivatives, and especially chitosan lactate; hyaluronic acid derivatives, such as the metal salts, and especially sodium hyaluronate; dextran and its derivatives, such as dextran sulphate; cellulose, hydroxyethylcellulose, hydroxypropylcellulose, polyquaterniums 10 and 24; starch, amylose and amylopectin and their derivatives; and chondroprotein and its derivatives, such as chondroitin and chondroitin sulphate.

11. Use according to Claim 10, for the preparation of a makeup remover composition which causes little or no irritation, and/or a composition which has a gentle feel, and/or a gelled composition.

12. Use according to one of Claims 10 to 11, in which the said compound is honey.

13. Use according to one of Claims 10 to 12, in which the polyholoside is present in the final composition in a quantity of from 0.01 to 5% by weight, preferably from 0.02 to 1% by weight, relative to the composition.

14. Use according to one of Claims 10 to 13, in which the composition does not comprise any surfactant.

15. Use according to one of Claims 10 to 14, in a composition in the form of an emulsion, an optionally gelled aqueous solution, a lotion, especially a biphase lotion, a cream, a milk or a mousse.

16. Method of cleansing and/or removing makeup from the skin and/or mucosae and/or eyes, characterized in that a composition according to any one of Claims 1 to 9 is applied to the skin and/or to the mucosae and/or to the eyes.

## Patentansprüche

1. Zusammensetzung zur Reinigung und/oder zum Abschminken der Haut und/oder der Schleimhäute und/oder der Augen, die ein homogenes Polyholosid und/oder eine Verbindung enthält, die ein homogenes Polyholosid enthält, das unter Chitin und seinen Derivaten, den Derivaten der Hyaluronsäure, den Derivaten von Dextran, Polyquaternium 10 und Polyquaternium 24, Amylose und Amylopectin und ihren Derivaten, Chondroid und seinen Derivaten ausgewählt ist.

2. Zusammensetzung nach Anspruch 1, wobei das homogene Polyholosid unter Chitosanderivaten, den Metallsalzen der Hyaluronsäure, Dextransulfat, Chondroitin und Chondroitinsulfat ausgewählt ist.

3. Zusammensetzung nach Anspruch 1 oder 2, wobei das homogene Polyholosid unter Chitosanlactat, Natriumhyaluronat ausgewählt ist.

4. Zusammensetzung nach Anspruch 1, wobei die obige Verbindung aus Honig besteht.

5. Zusammensetzung nach einem der vorhergehenden Ansprüche, wobei das Polyholosid in der fertigen Zusammensetzung in einem Mengenanteil von 0,01 bis 5 Gew.-% enthalten ist.

6. Zusammensetzung nach einem der vorhergehenden Ansprüche, wobei das Polyholosid in der fertigen Zusammensetzung in einem Mengenanteil von 0,02 bis 1 Gew.-%, bezogen auf die Zusammensetzung, enthalten ist.

7. Zusammensetzung nach einem der vorhergehenden Ansprüche, die keinen grenzflächenaktiven Stoff enthält.

8. Zusammensetzung nach einem der vorhergehenden Ansprüche, die in Form einer Emulsion, einer wäßrigen Lösung, die gegebenenfalls in ein Gel übergeführt ist, einer Lotion, insbesondere zweiphasigen Lotion, einer Creme, einer Milch, eines Schaums vorliegt.

9. Zusammensetzung nach einem der vorhergehenden Ansprüche, dadurch gekennzeichnet, daß sie in Form einer Einzeldosis verpackt ist.

10. Verwendung mindestens eines homogenen Polyholosids und/oder mindestens einer Verbindung, die ein homogenes Polyholosid enthält, als Reinigungsmittel oder Mittel zum Abschminken in einer Zusammensetzung zur Reinigung und/oder zum Abschminken der Haut und/oder der Schleimhäute und/oder der Augen, die einen kosmetisch oder dermatologisch akzeptablen Träger enthält, wobei das Polyholosid unter Chitin und seinen Derivaten, wie z.B. Chitosan-Derivaten und insbesondere Chitosanlactat, den Derivaten der Hyaluronsäure, wie z.B. den Metallsalzen und insbesondere Natriumhyaluronat, Dextran und seinen Derivaten, wie z.B. Dextransulfat, Cellulose, Hydroxyethylcellulose, Hydroxypropylcellulose, Polyquaternium 10 und Polyquaternium 24, Stärke, Amylose und Amylopectin und ihren Derivaten, Chondroid und seinen Derivaten, wie z.B. Chondroitin und Chondroitinsulfat, ausgewählt ist.

11. Verwendung nach Anspruch 10 zur Herstellung einer wenig oder nicht reizenden Abschmink-Zusammensetzung und/oder einer Zusammensetzung, die sich weich bzw. geschmeidig anfühlt und/oder einer Zusammensetzung, die in Gelform vorliegt.

12. Verwendung nach Anspruch 10 oder 11, wobei die Verbindung aus Honig besteht.

13. Verwendung nach einem der Ansprüche 10 bis 12, wobei das Polyholosid in der fertigen Zusammensetzung in einem Mengenanteil von 0,01 bis 5 Gew.-%, vorzugsweise 0,02 bis 1 Gew.-%, bezogen auf die Zusammensetzung, enthalten ist.

14. Verwendung nach einem der Ansprüche 10 bis 13, wobei die Zusammensetzung keinen grenzflächenaktiven Stoff enthält.

15. Verwendung nach einem der Ansprüche 10 bis 14 in einer Zusammensetzung, die in Form einer Emulsion, einer wäßrigen Lösung, die gegebenenfalls in ein Gel übergeführt ist, einer Lotion, insbesondere einer zweiphasigen Lotion, einer Creme, einer Milch, eines Schaums vorliegt.

16. Verfahren zur Reinigung und/oder zum Abschminken der Haut und/oder der Schleimhäute und/oder der Augen, dadurch gekennzeichnet, daß auf die Haut und/oder die Schleimhäute und/oder die Augen eine Zusammensetzung nach einem der Ansprüche 1 bis 9 aufgetragen wird.
